(19) Europäisches Patentamt
European Patent Office
Office européen des brevets

(11) Publication number: **0 234 668 B1**

# (12) EUROPEAN PATENT SPECIFICATION

(45) Date of publication of patent specification: **03.04.91**

(51) Int. Cl.5: **C07C 69/587**, C07C 67/00

(21) Application number: **87200327.2**

(22) Date of filing: **25.02.87**

## (54) Preparation of mixtures of octadienyl esters of nonatrienoic acids.

(30) Priority: **26.02.86 GB 8604789**

(43) Date of publication of application:
**02.09.87 Bulletin 87/36**

(45) Publication of the grant of the patent:
**03.04.91 Bulletin 91/14**

(84) Designated Contracting States:
**AT BE CH DE FR GB IT LI NL**

(56) References cited:
**EP-A- 0 050 445**
**GB-A- 2 003 875**

**JOURNAL OF THE CHEMICAL SOCIETY, CHEMICAL COMMUNICATIONS, vol. 2, no. 13, 7th July 1976, pages 605-606; Y. INOUE et al.: "Reaction of carbon dioxide with butadiene catalysed by palladium complexes. Synthesis of 2-Ethylidenehept-5-en-4-olide"**

**JOURNAL OF THE CHEMICAL SOCIETY, PERKIN TRANSACTIONS 1, 1980, pages 693-698; A. MUSCO "Co-oligomerization of Butadiene and carbon dioxide catalysed by tertiary phosphine-palladium complexes"**

(73) Proprietor: **SHELL INTERNATIONALE RESEARCH MAATSCHAPPIJ B.V.**
**Carel van Bylandtlaan 30**
**NL-2596 HR Den Haag(NL)**

(72) Inventor: **Drent, Eit**
**Badhuisweg 3**
**NL-1031 CM Amsterdam(NL)**

(74) Representative: **Aalbers, Onno et al**
**P.O. Box 302**
**NL-2501 CH The Hague(NL)**

**EP 0 234 668 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to a process for the preparation of octadienyl esters of nonatrienoic acids by reaction of 1,3-butadiene with carbon dioxide.

Octadienyl esters of nonatrienoic acids are valuable intermediate products in the preparation of lubricants, additives for lubricating oils, plasticizers for plastic materials, metal soaps, alkyl resins, detergents, emulsifying agents and cosmetic and pharmaceutical products.

It is known from US patent specification 4,167,513 that reaction of 1,3-butadiene with carbon dioxide yields octadienyl esters of nonatrienoic acids having the empirical formula $C_8H_{11}C(O)OC_8H_{13}$ and the lactone having the formula A

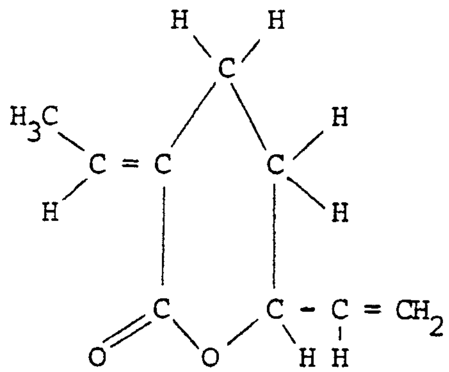

(A)

also referred to as "2-ethylidene-hepta-6-ene-5-olide", in the presence of a monodentate phosphine complex of palladium. It appears from the examples that dimerization of 1,3-butadiene to a mixture of octatrienes takes place as a side reaction and that said esters were obtained with a considerably lower selectivity than the octatrienes. The selectivity to a certain compound, expressed as a percentage, is defined herein as 100 × a/b, in which "a" is the amount of starting compound that has been converted into that certain compound and "b" is the total amount of starting compound that has been converted.

It is an object of the present invention to prepare octadienyl esters of nonatrienoic acids in a selectivity which is good and higher than that to octatrienes.

Accordingly, the invention provides a process for the preparation of octadienyl esters of nonatrienoic acids by reaction of 1,3-butadiene with carbon dioxide, which process is carried out in the presence of a solvent for the reactants and of a catalytic system prepared by combining:-

a) palladium and/or a palladium compound,

b) a bidentate ligand of the general formula I

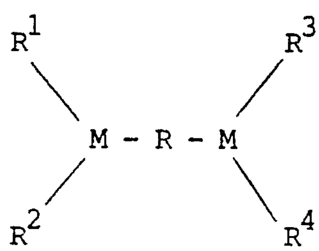

(I)

in which each M individually represents a phosphorus, arsenic or antimony atom, R represents a divalent organic bridging group having three carbon atoms in the bridge, none of these carbon atoms carrying substituents that may cause steric hindrance and $R^1$, $R^2$, $R^3$ and $R^4$ each individually represent an optionally substituted hydrocarbon group, and

c) an amine.

Surprisingly, the process according to the present invention allows octadienyl esters of nonatrienoic acids with a selectivity which can be more than twice than that to octatrienes.

Both homogeneous and heterogeneous catalytic systems can be used. Homogeneous systems are preferred. Suitable palladium compounds are salts of palladium with, for example, nitric acid, sulphuric acid or alkanoic acids, preferably those having not more than 12 carbon atoms per molecule. Salts of hydrohalogenic acids are also usable. Palladium acetate is particularly preferred. Palladium acetylacetonate

is another example of a palladium compound that can be used. Palladium on carbon and palladium bonded to an ion exchanger, for example one containing sulphonic acid groups, are examples of suitable heterogeneous palladium compounds.

Where, in the bidentate ligand, it is said that substituents offering steric hindrance should be absent, this means that no substituents may be present that are able to hinder the formation of complex compounds having the general formula B

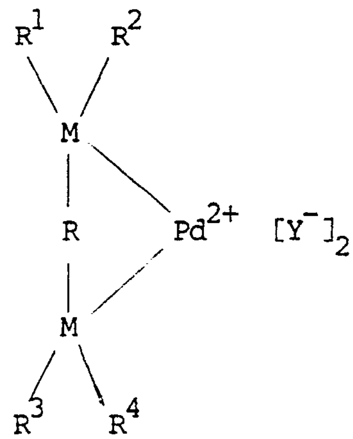

(B)

In formula B, Y represents a non-coordinating anion, whilst $Pd^{2+}$ can also be written as

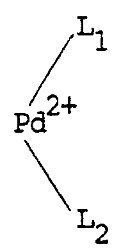

,

in which the ligands $L_1$ and $L_2$ are weakly coordinated solvent ligands, for example acetonitrile, methanol, acetone or acetylacetone, or correspond with those employed in the palladium compounds described in the preceding paragraph.

In the bidentate ligand of the general formula I, each M preferably represents a phosphorus atom. The hydrocarbon groups $R^1$, $R^2$, $R^3$ and $R^4$ will as a rule contain 2 to 18 carbon atoms, preferably 6 to 14 carbon atoms. The hydrocarbon groups may be alkyl, cycloalkyl or, which are preferred, aryl groups. The hydrocarbon groups may optionally be substituted, for example with halogen atoms, alkoxy groups - preferably those having not more than five carbon atoms - and cyano groups. The aryl group may be an anthryl (for example 1- or 2-anthryl), a 1- or 2-naphthyl or, which is preferred, a phenyl group.

Preferred bridging groups -R- are those having the formula II

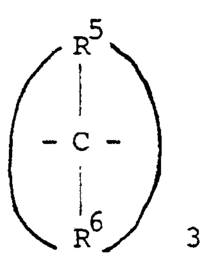

(II)

in which $R^5$ and $R^6$ each individually represent a hydrogen atom or an optionally substituted hydrocarbon group offering no steric hindrance. Substituents $R^5$ and $R^6$ preferably represent hydrogen atoms. The bridging group R may also make part of a cyclic structure, for example an aromatic or cycloaliphatic group; the carbon to carbon bonds in the bridge may be saturated or unsaturated and in the cyclic or non-cyclic groups attached to the bridge one or more hetero atoms, for example sulphur, oxygen, nitrogen or iron,

may have been substituted for carbon atoms. Very good results have been obtained with 1,3-di-(diphenylphosphino)propane. Other examples of suitable bidentate ligands are 1,3-di(di-p-tolylphosphino)-propane and 1,3-di(dimethylphosphino)propane. If desired, in addition a monodentate phosphine, for example triphenylphosphine or tri(p-chlorophenyl)phosphine, may also be present.

It has been found that amending the process according to the present invention by deleting the amine suppresses formation of octadienyl esters of nonatrienoic acids and increases the selectivity to octatrienes. Suitable amines which may be present include alkyl, cycloalkyl, aryl, alkaryl and aralkyl amines, preferably containing up to 30 carbon atoms per molecule, and amines in which the nitrogen atom is part of a heterocyclic ring, for example pyrrole, alkyl-substituted pyrroles, pyrrolidine, alkyl-substituted pyrrolidines, pyridine, alkyl-substituted pyridines, piperidines, alkyl-substituted piperidines, pyrimidine, alkyl-substituted pyrimidines, pyrazine, benztriazole, tetraethylenediamine, N,N′-dimethyltrimethylenediamine, 1,10-phenanthroline, alkyl-substituted 1,10-phenanthrolines, morpholine and alkyl-substituted morpholines. Very good results have been obtained with trialkylamines, particularly with those in which the alkyl groups do not have more than 5 carbon atoms, more particularly with triethylamine. Other examples of suitable amines are diethylamine, tripropylamine, tributylamine, tripentylamine and trihexylamine.

The following molar ratios are not critical and may vary within wide ranges, for example within those indicated hereinafter:-

| | |
|---|---|
| bidentate ligand of the general formula I to palladium and/or palladium compound | 0.1 to 10 |
| amine to palladium and/or palladium compound | 0.1 to 500 |

The molar ratio bidentate ligand of the general formula I to palladium and/or palladium compound is preferably in the range of from 0.9 to 2 and, more preferably, of from 0.9 to 1.1. The highest selectivity to octadienyl esters of nonatrienoic acids are usually obtained when this molar ratio is 1.0.

The molar ratio amine to palladium and/or palladium compound may be, for example, in the range of from 0.1 to 1, or of from 1 to 50, or of from 50 to 500.

The quantity of palladium and/or palladium compound is not critical and may vary within wide ranges. Preferably, quantities in the range of from $10^{-8}$ to $10^{-1}$ mol palladium compound per mol 1,3-butadiene are used. Usually, molar ratios carbon dioxide to 1,3-butadiene greater than 0.25, for example between 1 and 20 will be used.

The process according to the present invention can be carried out at a temperature and a pressure which are not critical and may vary within wide ranges. Preferably, a temperature in the range of from 20 °C to 200 °C, in particular 50 °C to 150 °C and a total pressure in the range of from 5 to 100 bar will be used. In general, reaction times between 1 and 20 hours will be adequate.

The process according to the invention should be carried out in the presence of a solvent for the reactants, i.e. for 1,3-butadiene and carbon dioxide. Examples of such solvents are hydrocarbons, such as hexane, octane, benzene, toluene, the three xylenes, ethylbenzene, cumene and cyclohexane; halogenated hydrocarbons, such as chloroform, 1,2-dichloroethane, perfluoroalkanes, chlorobenzene and the three dichlorobenzenes; sulphones such as diethyl sulphone, diisopropyl sulphone and tetrahydrothiophene 1,1-dioxide (also referred to as "sulfolane"); N,N-dialkyl-substituted amides such as N,N-dimethylformamide and N-methylpyrrolidone; esters such as methyl benzoate, ethyl acetate and amyl acetate; alcohols such as methanol, ethanol, 2-propanol and 1,4-butanediol; ethers such as diethyl ether, 3,6-dioxaoctane, methyl tert.-butyl ether, tetrahydrofuran, diisopropyl ether, 1,4-dioxane, 2,5,8-trioxanonane (also referred to as "diglyme"), diphenyl ether and anisole; ketones such as acetone, diethyl ketone, acetylacetone or methyl isobutyl ketone; nitro compounds such as nitrobenzene, and nitriles, such as benzonitrile propionitrile and acetonitrile. Very good results have been obtained with acetonitrile. The amine which is present in the catalytic system may also be used as a solvent.

The process according to the invention can be carried out batchwise, semi-continuously or continuously.

The following examples further illustrate the invention.

Example 1

A magnetically stirred 300 ml autoclave was charged with acetonitrile (50 ml), palladium(II) acetate (0.5 mmol), 1,3-di(diphenylphosphino)propane (0.5 mmol), triethylamine (2 ml, 14.5 mmol) and 1,3-butadiene (7 ml, 80 mmol). Then, carbon dioxide was added until a total pressure of 30 bar was reached. The autoclave was heated for 5 h at a temperature of 80 °C.

Analysis of the reaction product by means of gas-liquid mass spectroscopy showed that the conversion of 1,3-butadiene was 55%, with the following selectivities:-

| | |
|---|---|
| octadienyl esters of nonatrienoic acids | 68% |
| lactone having the formula A | 7% |
| octatrienes | 24% |

Comparative Experiment 1

Example 1 was repeated with the exception that no triethylamine was present and that a temperature of 90 °C was used. The conversion of 1,3-butadiene was 70%, with a selectivity to octatrienes of 100%.

Comparative Experiment 2

Example 1 was repeated with the exception that 1,3-di(diphenylphosphino)propane (0.5 mmol) was replaced with 1,2-di(diphenylphosphino)ethane (0.5 mmol). The conversion of 1,3-butadiene was 40%, with the following selectivities:-

| | |
|---|---|
| octadienyl esters of nonatrienoic acids | 20% |
| lactone having the formula A | 14% |
| octatrienes | 65% |

Comparative Experiment 3

Example 1 was repeated with the exception that 1,3-di(diphenylphosphino)propane (0.5 mmol) was not present. No reaction was observed after 5h at 80 °C.

Comparative Experiment 4

Example 1 was repeated with the exception that 1,3-di(diphenylphosphino)propane (0.5 mmol) was replaced with 1,4-di(diphenylphosphino)butane (0.5 mmol).

The conversion of 1,3-butadiene was 30%, with a selectivity to octadienyl esters of nonatrienoic acids of 19% and to octatrienes of 80%.

Example 2

Example 1 was repeated with the exception that not 0.5 mmol but 1.0 mmol of 1,3-di-(diphenylphosphino)propane was used, the molar ratio 1,3-di(diphenylphosphino)propane to palladium(II) acetate being 2, and that the temperature was kept at 100 °C.

The conversion of 1,3-butadiene was 45%, with the following selectivities:-

| | |
|---|---|
| octadienyl esters of nonatrienoic acids | 45% |
| lactone having the formula A | 5% |
| octatrienes | 49% |

Comparison with Example 1 shows that increasing the molar ratio 1,3-di(diphenylphosphino)propane to palladium(II) acetate from 1.0 to 2.0 decreases the selectivity to octadienyl esters of nonatrienoic acids and renders the catalytic system less active.

Example 3

Example 1 was repeated with the exception that triethylamine (2 ml) was replaced with N,N′-dimethyltrimethylenediamine (1 ml).

The conversion of 1,3-butadiene was 30%, with the following selectivities:-

| | |
|---|---|
| octadienyl esters of nonatrienoic acids | 49% |
| lactone having the formula A | 2% |
| octatrienes | 49% |

Comparison with Example 1 shows that the use of triethylamine allows a higher selectivity to the esters and renders the catalytic system more active.

## Claims

1. Process for the preparation of mixtures of octadienyl esters of nonatrienoic acids by reaction of 1,3-butadiene with carbon dioxide, which process is carried out in the presence of a solvent for the reactants and of a catalytic system prepared by combining:-
   a) palladium and/or a palladium compound,
   b) a bidentate ligand of the general formula I

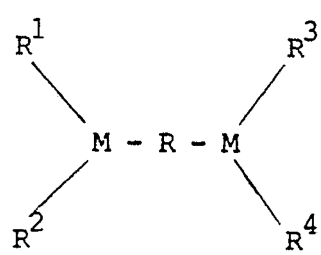

(I)

   in which each M individually represents a phosphorus, arsenic or antimony atom, R represents a divalent organic bridging group having three carbon atoms in the bridge, none of these carbon atoms carrying substituents that may cause steric hindrance and $R^1$, $R^2$, $R^3$ and $R^4$ each individually represent an optionally substituted hydrocarbon group, and
   c) an amine.

2. Process as claimed in claim 1 in which the catalytic system is homogeneous.

3. Process as claimed in claim 1 or 2 in which the palladium compound is a palladium salt.

4. Process as claimed in claim 3 in which the palladium salt is palladium acetate.

5. Process as claimed in any one of the preceding claims in which the hydrocarbon groups $R^1$, $R^2$, $R^3$ and

$R^4$ each individually represent an optionally substituted aryl group having in the range of from 6 to 14 carbon atoms.

6. Process as claimed in claim 5 in which the aryl group is a phenyl group.

7. Process as claimed in claim 6 in which the bidentate ligand is 1,3-di(diphenylphosphino)propane.

8. Process as claimed in any one of the preceding claims in which the amine is a trialkylamine.

9. Process as claimed in claim 8 in which the alkyl groups in the amine do not have more than 5 carbon atoms.

10. Process as claimed in claim 9 in which the amine is triethylamine.

11. Process as claimed in any one of the preceding claims in which a molar ratio bidentate ligand of the general formula I to palladium and/or palladium compound in the range of from 0.9 to 2 is used.

12. Process as claimed in any one of the preceding claims which is carried out at a temperature in the range of from 20 °C to 200 °C.

13. Process as claimed in any one of the preceding claims which is carried out at a total pressure in the range of from 5 to 100 bar.

14. Process as claimed in any one of the preceding claims in which acetonitrile is used as a solvent.

## Revendications

1. procédé de préparation de mélanges d'esters octadiéniques d'acides nonatriénoïques par réaction du 1,3-butadiène avec l'anhydride carbonique, procédé qui est mis en oeuvre en présence d'un solvant pour les corps en réaction et d'un système catalytique préparé en combinant :
   a) du palladium et/ou un composé du palladium,
   b) un ligand bidenté de la formule générale I

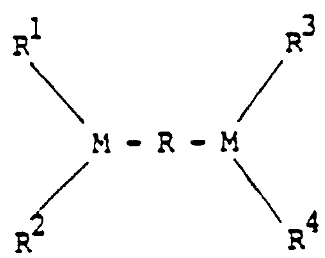

(I)

   où chaque M individuellement représente un atome de phosphore, d'arsenic ou d'antimoine, R représente un groupe organique divalent formant pont ayant trois atomes de carbone dans le pont, aucun de ces atomes de carbone ne portant de substituants capables de causer un empêchement stérique et $R^1$, $R^2$, $R^3$ et $R^4$ représentent chacun individuellement un groupe d'hydrocarbure éventuellement substitué, et
   c) une amine.

2. Procédé selon la revendication 1, dans lequel le système catalytique est homogène.

3. Procédé selon la revendication 1 ou 2, dans lequel le composé du palladium est un sel de palladium.

4. Procédé selon la revendication 3, dans lequel le sel de palladium est de l'acétate de palladium.

5. Procédé selon l'une quelconque des revendications précédentes, dans lequel les groupes d'hydrocarbures $R^1$, $R^2$, $R^3$ et $R^4$ représentent chacun individuellement un groupe aryle éventuellement substitué

ayant de 6 à 14 atomes de carbone.

**6.** Procédé selon la revendication 5, dans lequel le groupe aryle est un groupe phényle.

**7.** Procédé selon la revendication 6, dans lequel le ligand bidenté est du 1,3-di(diphénylphosphino)-propane.

**8.** Procédé selon l'une quelconque des revendications précédentes, dans lequel l'amine est une trialcoylamine.

**9.** Procédé selon la revendication 8, dans lequel les groupes alcoyle dans l'amine n'ont pas plus de 5 atomes de carbone.

**10.** Procédé selon la revendication 9, dans lequel l'amine est la triéthylamine.

**11.** Procédé selon l'une quelconque des revendications précédentes, dans lequel on utilise un rapport molaire du ligand bidenté de la formule générale I au palladium et/ou au composé du palladium compris entre 0,9 et 2.

**12.** Procédé selon l'une quelconque des revendications précédentes, qui est mis en oeuvre à une température comprise entre 20° C et 200° C.

**13.** Procédé selon l'une quelconque des revendications précédentes, qui est mis en oeuvre à une pression totale comprise entre 5 et 100 bars.

**14.** Procédé selon l'une quelconque des revendications précédentes, dans lequel on utilise l'acétonitrile comme solvant.

## Ansprüche

**1.** Verfahren zur Herstellung von Gemischen von Octadienylestern von Nonatriencarbonsäuren durch Umsetzung von 1,3-Butadien mit Kohlendioxid, welches Verfahren in Anwesenheit eines Lösungsmittels für die Reaktanten und eines katalytischen Systems ausgeführt wird, das durch Kombinieren von:
a) Palladium und/oder einer Palladiumverbindung,
b) einem Bidentatliganden der allgemeinen Formel I

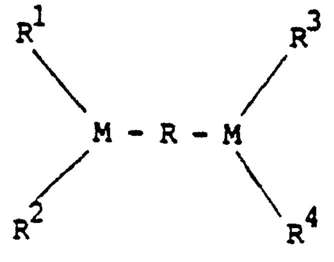

(I) ,

worin jedes M unabhängig voneinander ein Phospor-, Arsen-oder Antimonatom darstellt, R eine zweiwertige organische Brückengruppe mit drei Kohlenstoffatomen in der Brücke bedeutet, wobei keines dieser Kohlenstoffatome Substituenten trägt, die eine sterische Behinderung verursachen könnten, und $R^1$, $R^2$, $R^3$ und $R^4$ jeweils unabhängig voneinander eine gegebenenfalls substituierte Kohlerwasserstoffgruppe bedeuten, und
c) einem Amin bereitet worden ist.

**2.** Verfahren nach Anspruch 1, worin das katalytische System homogen ist.

**3.** Verfahren nach Anspruch 1 oder 2, worin die Palladiumverbindung ein Palladiumsalz ist.

**4.** Verfahren nach Anspruch 3, worin das Palladiumsalz Palladiumacetat ist.

5. Verfahren nach einem der vorstehenden Ansprüche, worin die Kohlenwasserstoffgruppen $R^1$, $R^2$, $R^3$ und $R^4$ jeweils unabhängig voneinander eine gegebenenfalls substituierte Arylgruppe mit 6 bis 14 Kohlenstoffatomen bedeuten.

6. Verfahren nach Anspruch 5, worin die Arylgruppe eine Phenylgruppe ist.

7. Verfahren nach Anspruch 6, worin der Bidentatligand 1,3-Di(diphenyl-phosphino)propan ist.

8. Verfahren nach einem der vorstehenden Ansprüche, worin das Amin ein Trialkylamin ist.

9. Verfahren nach Anspruch 8, worin die Alkylgruppen in dem Amin nicht mehr als 5 Kohlenstoffatome enthalten.

10. Verfahren nach Anspruch 9, worin das Amin Triethylamin ist.

11. Verfahren nach einem der vorstehenden Ansprüche, worin ein Molverhältnis von Bidentatligand der allgemeinen Formel I zu Palladium und/oder Palladiumverbindung im Bereich von 0.9 bis 2 angewendet wird.

12. Verfahren nach einem der vorstehenden Ansprüche, welches bei einer Temperatur im Bereich von 20° C bis 200° C ausgefürt wird.

13. Verfahren nach einem der vorstehenden Ansprüche, welches bei einem Gesamtdruch im Bereich von 5 bis 100 bar ausgeführt wird.

14. Verfahren nach einem der vorstehenden Ansprüche, worin Acetonitril als Lösungsmittel verwendet wird.